# EUROPEAN PATENT APPLICATION

(11) **EP 0 669 107 A1**
(43) Date of publication of application: **30.08.1995**
(21) Application number: 95301067.5
(22) Date of filing: 20.02.1995
(51) Int. Cl.: A61B 17/36, A61B 17/32

(54) **Surgical Instrument with attached laser instrument**

(30) Priority: 23.02.1994 US 200680
(71) Applicant: SMITH & NEPHEW DYONICS INC, Andover, Massachusetts 01810 (US)
(72) Inventor: Federico, Charles W., Westford, Massachusetts 01886 (US); Santamaria, Carl J., Tyngsboro, Massachusetts 01879 (US); Lucey, Paul V., Sandown, New Hampshire 03873 (US); Kennedy, John E., Lowell, Massachusetts 01852 (US)
(74) Representative: Gilholm, Stephen Philip

(57) **Abstract**

A surgical device (10) in which a laser instrument for applying laser energy to body tissue is attached to a surgical instrument (16) that performs a surgical operation on body tissue. A mounting assembly (20) for attaching the instruments together allows the laser instrument to be moved between a retracted position in which the tip (140) of the laser instrument (which emits laser energy for, e.g, cauterization) is disposed proximally of a surgical implement on the surgical instrument, and an extended position in which the laser tip is disposed distally of the surgical implement. The mounting assembly also permits the laser instrument to be rotated with respect to the surgical instrument, and clamps the laser instrument in any selected rotational orientation and axial position with respect to the surgical instrument. The mounting assembly is threaded for easy attachment to a corresponding set of threads on the surgical instrument.

## Description

This invention relates to surgical instruments, such as arthroscopic surgical instruments.

Several different kinds of surgical instruments have been developed for performing arthroscopic and other endoscopic surgical procedures. Some of these surgical instruments are powered, that is, operated by a motor; others are manual. Motor-driven instruments typically are received by a handpiece which houses the motor. Manual instruments are operated with a trigger-like handle. The instruments may include a wide variety of surgical implements for performing different types of surgical operations on body tissue. For example, some instruments are equipped with blades for cutting soft tissue, while others have burrs for abrading bone tissue. Still other instruments (such as forceps and graspers) grip, rather than cut, tissue. Electrocautery instruments may also be used during arthroscopy.

A typical cutting or abrading arthroscopic surgical instrument includes a stationary outer tube within which an inner tube is moved (either manually or driven by a motor) during operation. The surgical implement is mounted to the distal end of the inner tube. Tissue or bone is exposed to the surgical implement through an opening in the distal end of the outer tube, and tissue or bone fragments cut by the moving implement are drawn through the interior of the inner tube by suction applied at the proximal end of the instrument. Examples of powered surgical instruments are described in U.S. Patent Nos. 4,203,444, 4,274,414, 4,834,729, 4,842,578, and 4,705,038; examples of manual surgical instruments are described in U.S. Patent Nos. 4,522,206 and 4,662,371. All of these patents are assigned to the present assignee and are incorporated herein by reference.

It is often useful to introduce a laser instrument into the body during arthroscopy or other surgical procedures. Laser energy applied by the laser instrument is used to, e.g., ablate tissue or cauterize bleeding vessels.

This invention, in one general aspect, features a surgical device in which a laser instrument for applying laser energy to body tissue is attached to a surgical instrument that performs a surgical operation on body tissue. In a related aspect, the invention features a mounting assembly for attaching the laser instrument to the surgical instrument.

Among other advantages, the invention allows the surgical instrument and the laser instrument to be introduced into the body together as a single surgical device (for example, through the same cannula during arthroscopy). This eliminates the need to replace one of the instruments with the other when either surgical operation (such as cutting, abrading, or grasping body tissue) or laser operation (e.g., cauterization) is to be performed. Put another way, the invention provides a unitary surgical device that places both the surgical instrument and the laser instrument at the user's fingertips for use on demand. In addition to simplifying the surgical procedure, the invention reduces the trauma to the patient that accompanies repeated insertions and withdrawals of separate instruments in the body.

Preferred embodiments include the following features.

The laser instrument is mounted for movement between a retracted position in which the laser instrument is disposed proximally of a surgical implement on the surgical instrument that performs the surgical operation (e.g., cutting tissue), and an extended position in which a tip of the laser instrument (which emits the laser energy) is disposed distally of the surgical implement. This affords the user with precise control of the surgical tools -- when the laser instrument is not needed, it is simply retracted to reduce the possibility that it will interfere with the operation of the surgical implement. Likewise, extending the tip of the laser instrument beyond the surgical implement allows the lasing tip to be positioned closely adjacent the tissue to be operated on, without interference from the surgical implement.

The mounting assembly also permits relative rotation between the laser instrument and the surgical instrument. A clamp secures the laser instrument in any selected rotational orientation with respect to the surgical instrument. This feature provides the user with still more flexibility in positioning the instruments.

The mounting assembly receives a portion of the laser instrument and is constructed to releasably engage the surgical instrument. One example of this construction is discussed below.

The mounting assembly includes an elongated cannula within which the surgical instrument and the laser instrument are disposed. The cannula has an open distal end through which the surgical implement and the tip of the laser instrument may protrude. An actuator selectively moves the laser instrument axially with respect to the cannula between a retracted position and an extended position. In the extended position, the tip of the laser instrument extends distally of the open end of the cannula; in the retracted position, it does not.

The cannula extends distally from a frame, within which a proximal end of the laser instrument is disposed. The actuator moves the proximal end of the laser instrument axially in the frame, thereby moving the laser instrument tip axially between the retracted and extended positions. The surgical instrument is larger in diameter than the laser instrument, and the cannula has regions of different diameters along its length to accommodate the differently-sized instruments. The rotational feature of the mounting assembly allows the cannula to be rotated to any selected orientation with respect to the surgical implement to assist the user in inserting the surgical device into the body.

The actuator includes a knob attached to the proximal end of the laser instrument and extending through a slot in the frame. The slot includes enlarged openings that respectively correspond to the retracted and extended positions. A spring resiliently urges the knob toward the frame to seat the knob within one of the openings when the retracted or extended position is reached. In addition to giving the user tactile feedback as to the relative positions of the instrument, this feature helps retain the laser instrument in the retracted or extended position.

The mounting assembly also includes a coupling for attaching the cannula to the surgical instrument. A base on the coupling receives a stem attached to the frame and the cannula, and allows the cannula and the laser instrument to be moved axially with respect to the surgical instrument as well as rotated with respect to the surgical instrument. In this way, the cannula may be moved axially with respect to the base between a first position, in which the surgical implement extends through the open end of the cannula, and a second position, in which the open end of the cannula surrounds the surgical implement. The clamp secures the stem within the base at a selected axial position and in a selected rotational orientation with respect to the surgical instrument.

It thus will be appreciated that the surgical device has three operative configurations. In the first configuration, both the cannula and the tip of the laser instrument are retracted with respect to the surgical implement (i.e., the cannula is in the first position, and the laser instrument is in the retracted position). The surgical instrument can thus be used with little risk of interference from the laser instrument. In the second configuration, the cannula is extended (i.e., to the second position) while the laser instrument remains in the retracted position. As a result, the cannula encloses both the surgical implement and the laser tip to, e.g., protect the instruments when inserting the surgical device into the body. In the third configuration, the laser instrument is moved to the extended position with the cannula in the second position. The extended cannula helps protect the surgical implement during laser operation.

The invention is particularly (though by no means exclusively) suitable for use with surgical instruments that are threaded to receive an accessory -- the mounting assembly of the invention is compatibly threaded for easy attachment to the surgical instrument. One example of such a surgical instrument includes an elongated tube that extends from a base and carries the surgical implement. The surgical instrument base has an annular collar radially spaced from the elongated tube. A set of threads are disposed on the interior of the collar. Such surgical instruments are commercially available from Smith & Nephew Dyonics Inc. of Andover, Massachusetts. The base of the coupling includes a threaded proximal end configured to fit radially between the collar and the elongated member and engage the threads.

Preferably, the surgical instrument is a powered arthroscopic surgical instrument (that is, an instrument constructed to be driven by a motor, rather than by hand). But the invention may also be used with manual instruments, as well as with other types of surgical instruments.

Other features and advantages of the invention will become apparent from the following detailed description, and from the claims.

Fig. 1 shows a cross-sectioned view of a surgical device in which a laser instrument is mounted to a surgical instrument.

Fig. 2 is a cross-sectional view of a mounting assembly for mounting the laser instrument to the surgical instrument.

Fig. 2a is a cross-sectional view of the mounting assembly taken along section a-a of Fig. 2.

Fig. 3 is a bottom view of the mounting assembly of Fig. 2.

Fig. 4 is a cross-sectional view of the laser instrument.

Fig. 5 is an enlarged, cross-sectional view of portions of the surgical instrument, the laser instrument, and the mounting assembly.

Figs. 6-8 are useful in understanding the operation of the surgical device of Fig. 1.

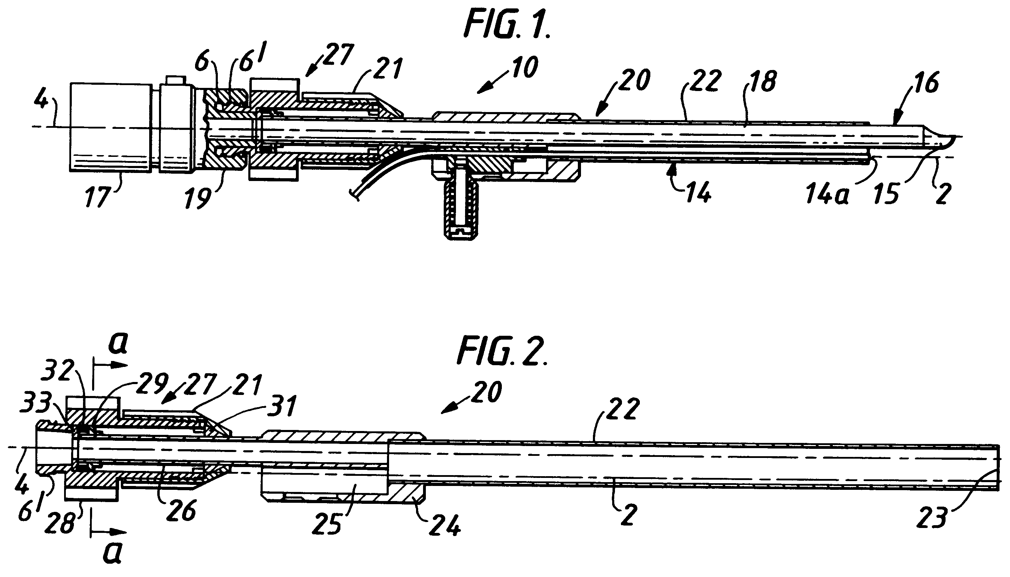

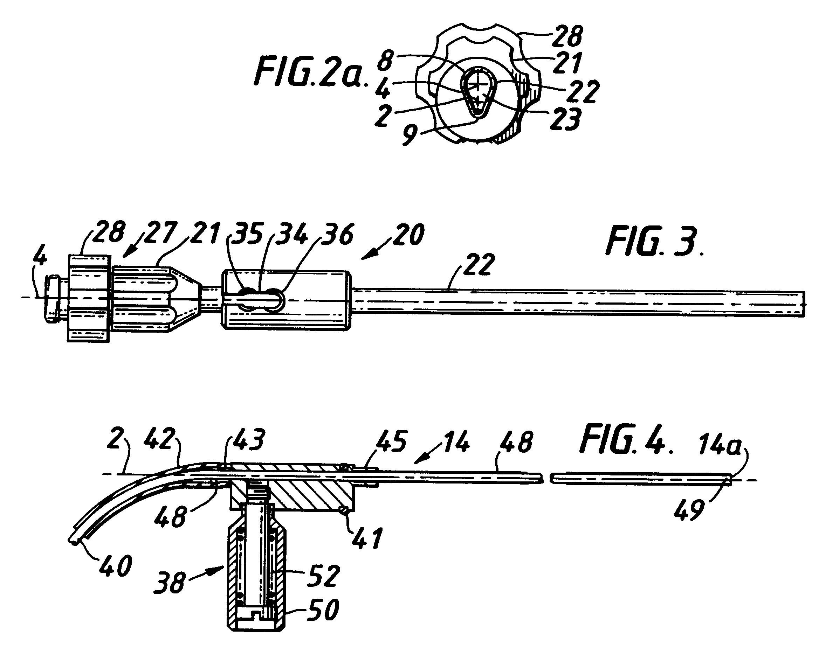

Referring to Fig. 1, surgical device 10, suitable for performing, for example, closed, arthroscopic surgery on the knee, includes laser instrument 14 attached to a surgical instrument 16 by a mounting assembly 20 (also shown in Fig. 2). Surgical instrument 16 supports on its distal end a surgical implement 15 suitable for performing a surgical operation on body tissue (such as cutting, abrading, or grasping the tissue). Laser instrument 14 emits laser energy from distal tip 14a to perform laser operations on body tissue (e.g., ablating tissue or cauterizing bleeding vessels).

As described in detail below, mounting assembly 20 is constructed to allow laser instrument 14 to be moved axially along its centerline 2 with respect to surgical instrument 16. This enables laser instrument 14 to be extended distally of surgical implement 15 during laser operation and thereafter retracted behind surgical implement 15. In addition, mounting assembly 20 permits laser instrument 14 to be rotated with respect to surgical instrument 16 about centerline 4 of surgical instrument 16. A clamp 21 of mounting assembly 20 is engaged to secure laser instrument 14 in any selected axial position and rotational orientation with respect to surgical instrument 16, as will be explained.

Surgical instrument 16 may have any suitable construction for performing the desired surgical operation. Surgical instrument 16 shown in the figures is a powered device -- that is, surgical instrument 16 is constructed to be received by a handpiece (not shown) that contains a motor for actuating surgical implement 15. Several such surgical instruments 16 are discussed in the aforementioned U.S. patents, and thus their details will not be repeated here. Suffice it to say that such surgical instruments 16 include a stationary outer tube 18 attached to a base 17 that fits within the handpiece. Surgical implement 15 is disposed on the distal end of outer tube 18 and coacts with an element disposed on the distal end of an inner tube (not shown) that extends through outer tube 18 and base 17. The inner tube is moved (e.g., rotated) by the motor to operate surgical implement 15.

Surgical implement 15 shown in the figures includes a cutting blade for severing tissue. It will be appreciated that surgical implement 15 will have a different configuration if other surgical operations (e.g., abrading, grasping) are to be performed.

Base 17 of surgical instrument 16 includes has an annular collar 19 that is radially spaced from outer tube 18. Threads 6 are formed on the inner surface of collar 19 for receiving any compatibly threaded accessory. A coupling 27 on the proximal end of mounting assembly 20 is provided with threads 6' that are compatible with threads 6. This feature enables mounting assembly 20 to be easily attached to and removed from surgical instrument 16 (and used with any compatibly-configured surgical instrument).

Referring to Figs. 2 and 3, mounting assembly 20 is shown removed from laser instrument 14 and surgical instrument 16. Mounting assembly 20 includes elongated cannula 22 that receives laser instrument 14 and outer tube 18 of surgical instrument 16. Cannula 22 has an open distal end 23 through which laser tip 14a and surgical implement 15 can protrude.

As can be seen from Fig. 1, surgical instrument 16 is larger in diameter than laser instrument 14. As shown in Fig. 2a, cannula 22 is in the shape of a teardrop in cross section to accommodate the different diameters of instruments 14, 16. The top portion (in Fig. 2a) of cannula has major radius 8 along the length of cannula 22 corresponds to the size of surgical instrument 16. The center of major radius 8 is on centerline 4 of surgical instrument 16. The lower portion of cannula 22 has a smaller, minor radius 9 corresponding to the size of laser instrument 14. The center of minor radius 9 is on laser instrument centerline 2. The teardrop shape of cannula 22 enables laser instrument 14 and surgical instrument 16 to maintain their positions within the major radius 8 and minor radius 9, respectively, when the user axially moves or rotates cannula 20 (and thus laser instrument 14) with respect to surgical instrument 16.

Rigidly secured (such as by brazing or welding) to the proximal end of cannula 22 is a hollow frame 24 having a cavity 25 for receiving the proximal end of laser instrument 14 (see Fig. 1). An elongated, tubular stem 26 is likewise rigidly secured to the proximal end of frame 24 and is received by coupling 27. More specifically, stem 26 is slidably and rotatably received within a cavity of a base 28 of coupling 27. The axial movement of stem 26 within base 28 is limited proximally by the engagement of an enlarged retainer 29 on stem 26 with a lip 33 on base 28. A ferrule 31 held in position at the distal end of the cavity by a clamping knob 21 limits the axial movement of retainer 29 distally, and thus serves to prevent stem 26 from being slid out of base 28. An o-ring 32 seals the interface between base 28 and retainer 29.

Knob 21 and ferrule 31 combine to form a clamp for holding stem 26 (and thus laser instrument 14) in any selected axial and rotational position with respect to surgical instrument 16. When knob 21 is turned in a counter-clockwise direction, ferrule 31 loosely contacts stem 26. This enables the user to rotate cannula 22 and laser instrument 14 with respect to base 28 and slide cannula 22 and laser instrument 14 axially toward or away from base 28. When cannula 22 and laser instrument 14 have been rotated and moved axially as desired, knob 21 is turned in a clockwise direction to squeeze ferrule 31 onto stem 26. As a result, stem 26 (and thus cannula 22 and frame 24) is held in position both axially and rotationally. Knob 21 includes ridges on its outer surface (Fig. 3) for ease of rotation.

The proximal end of base 28 includes threads 6' for engaging threads 6 of surgical instrument 16, as discussed above. Ridges are formed on the outer surface of base 28 (Fig. 3) to assist the user in attaching base 28 to base 19 of surgical instrument 16.

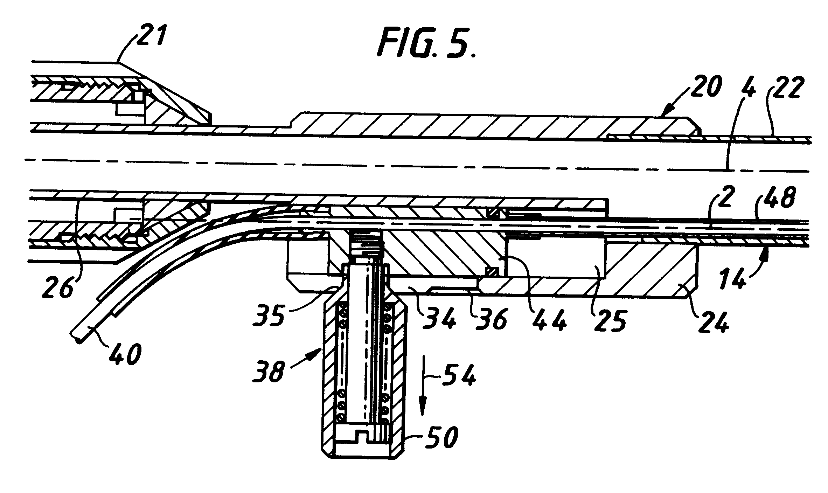

As shown in Figs. 2 & 3, frame 24 includes a slot 34 with two enlarged holes 35, 36 disposed at each end. Slot 34 and holes 35, 36 are used to secure and position actuator 38 (see Fig. 4) of laser instrument 14 within cavity 25, as will be discussed further below.

Referring to Fig. 4, laser instrument 14 includes laser fiber 40, strain relief tube 42, laser body 44, and laser tube 48. Laser light used during the surgical procedure travels to laser instrument 14 via laser fiber 40. Laser fiber 40 enters the proximal end of laser instrument 14 through a strain relief tube 42. Strain relief tube 42 is preferably made of resilient rubber (e.g., silicone) so that it is flexible, yet strong, to protect laser fiber 40 as it enters laser body 44.

Laser body 44 has an entrance tube 43 at its proximal end and an exit tube 45 at its distal end. The distal end of strain relief tube 42 snugly fits over entrance tube 43 of laser body 44. Additionally, entrance tube 43 has flange 48 which engages strain relief tube 42 with entrance tube 43. Laser tube 48 is attached to exit tube 45 by, for example, either adhesives, brazing, or welding.

Laser fiber 40 extends through laser tube 48 and exits laser tube 48 at the distal end 49 of laser instrument 14, thereby forming lasing tip 14a. Laser body 44 includes an o-ring 41 to provide a seal between laser instrument 14 and mounting assembly 20 at laser body 44 and frame 24.

Actuator 38 is used to position laser instrument 14 axially with respect to mounting assembly 20 and surgical instrument 16 along centerline 2. This positioning enables laser instrument 14 to be in either a retracted position, in which the laser tip 14a does not extend distally of cannula 22, or an extended position, in which laser tip 14a extends distally of the distal end of cannula 22.

Referring also to Fig. 5, actuator 38 includes knob 50 resiliently urged against frame 24 by a spring 52. Knob 50 is knurled to provide a gripping surface for the user. Laser instrument 14 is placed with cavity 25 of frame 24 by positioning laser instrument 14 within slot 34 while knob 50 is pulled down in the direction of arrow 54. Laser body 44 is then slid axially into cavity 25.

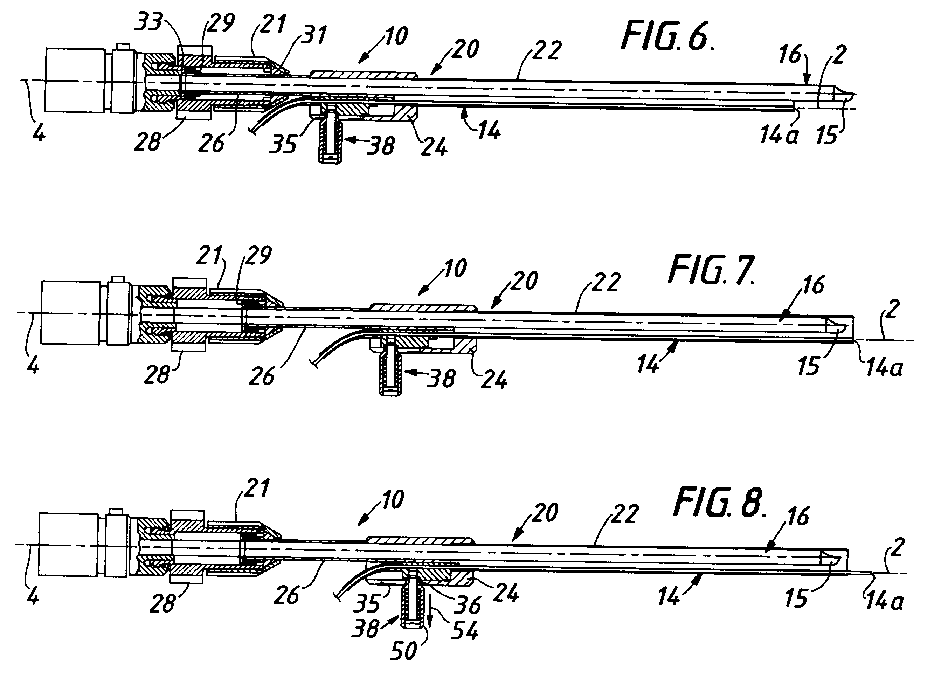

Frame 24, with holes 35, 36, enables laser instrument 14 to be moved axially with respect to cannula 22 and surgical instrument 16 into two secured positions. For example, when actuator 38 is seated in hole 35, laser instrument 14 is in the retracted position with respect to cannula 22 (see Figs. 6 and 7). The axial position of laser instrument 14 is changed by pulling down on knob 50, in the direction of arrow 54, and axially moving laser instrument 14 distally along centerline 2 until actuator 38 becomes seated in hole 36. When actuator 38 is positioned in hole 36, laser instrument 14 is in the extended position with respect to cannula 22 (shown in Fig. 8). Spring 52 helps maintain actuator 38 seated in respective holes 35, 36.

Figs. 6-8 show surgical device 10 in the different operational positions as used during a surgical procedure. Fig. 6 shows surgical device 10 with laser instrument 14 in the retracted position. In this configuration, laser instrument 14 is protected within cannula 22, and surgical implement 16 protrudes distally through open end 23 of cannula 22. Thus, in use, laser instrument 14 does not interfere with the operation of surgical instrument 16. In the retracted mode, actuator 38 rests in hole 35 of frame 24 (Fig. 5). Additionally, frame 24 is in its full proximal position, such that retainer 29 abuts lip 33 (of course, retainer 29 may be disposed forward of lip 33, so long as cannula 22 does not cover surgical implement 15).

Fig. 7 shows surgical device 10 in an intermediate or "rest" configuration, in which laser instrument 14 and surgical instrument 16 are both surrounded by the distal region of cannula 22. The position of laser instrument 14 has not changed relative to frame 24. Surgical instrument 16 becomes protected within cannula 22 by moving cannula 22 distally such that retainer 29 rests against ferrule 31. This configuration is particularly useful when surgical device 10 is being inserted into and removed from the body.

Fig. 8 shows surgical device 10 in the extended configuration, in which laser tip 14a extends distally beyond open end 23 of cannula 22. Surgical instrument 16 remains within cannula 22, and thus does not interfere with the operation of laser instrument 14. Laser instrument 14 is positioned to extended mode by pulling on knob 50, in the direction of arrow 54, and moving laser instrument axially along centerline 2, in the distal direction, until actuator 38 rests in hole 36. The position of cannula 22 with respect to base 28 does not change between the rest configuration and the extended configuration.

As discussed previously, the rotational position of laser instrument 14 relative to surgical instrument 16 may be changed by rotating stem 26 within base 28. Specifically, rotating stem 26 causes frame 24, cannula 22, and laser instrument 14 to rotate about centerline 4, and thus, also about surgical instrument 16. Knob 21 and ferrule 31, as discussed previously, are used to selectively secure and release stem 26 for axial and rotational movement.

Other embodiments are within the scope of the following claims.

For example, other types of surgical instruments may be used. The surgical instruments may be manual, rather than powered. The surgical instrument need not be a mechanical device. For example, an electrocautery instrument may be used as surgical instrument 16.

## Claims

1. A surgical device comprising
a surgical instrument for performing a surgical operation on body tissue,
a laser instrument for applying laser energy to body tissue, and
a mounting assembly for attaching said laser instrument to said surgical instrument.

2. The surgical device of claim 1 wherein said surgical instrument includes a surgical implement on a distal end thereof for performing the surgical operation, and said laser instrument includes a distal tip for emitting the laser energy, said mounting assembly comprising means for selectively moving said laser instrument between a retracted position in which said tip of said laser instrument is disposed proximally of said surgical implement, and an extended position in which said tip of said laser instrument is disposed distally of said surgical implement.

3. The surgical device of claim 1 or 2 wherein said mounting assembly includes means for allowing relative rotation between said laser instrument and said surgical instrument.

4. The surgical device of claim 3 further comprising a clamp for securing said laser instrument in a selected rotational orientation with respect to said surgical instrument.

5. The surgical device of claim 1 wherein said mounting assembly receives a portion of said laser instrument and includes means for releasably engaging said surgical instrument.

6. The surgical device of claim 1 wherein said surgical instrument includes a surgical implement on a distal end thereof for performing the surgical operation and said laser instrument includes a distal tip for emitting the laser energy, said mounting assembly comprising
an elongated cannula within which said surgical instrument and said laser instrument are disposed, said cannula having an open distal end through which said surgical implement and said tip of said laser instrument may protrude, and
an actuator for selectively moving said laser instrument axially within said cannula between a retracted position in which said tip of said laser instrument does not extend distally of said open end of said cannula, and an extended position in which said tip of said laser instrument extends distally of said open end of said cannula.

7. The surgical device of claim 6 further comprising a frame secured to a proximal end of said cannula, a proximal end of said laser instrument being disposed in said frame, said actuator being coupled to said proximal end to move said proximal end axially in said frame and cause said tip of said laser instrument to move axially within said cannula between said retracted position and said extended position.

8. The surgical device of claim 7 wherein said actuator includes a knob that is attached to said proximal end of said laser instrument and extends through a slot in said frame.

9. The surgical device of claim 8 wherein said slot includes enlarged openings that respectively correspond to said retracted position and said extended position, said actuator further comprising a spring for resiliently urging said knob toward said frame and causing a portion of said knob to seat within one of said openings, thereby to retain said laser instrument in the corresponding retracted position or extended position.

10. The surgical device of claim 6 wherein said surgical instrument has a larger diameter than that of said laser instrument, said cannula including regions of different diameters along its length for accommodating said surgical instrument and said laser instrument.

11. The surgical device of claim 6 wherein said mounting assembly further comprises a coupling for attaching said cannula to said surgical instrument, said coupling being constructed to allow said cannula and said laser instrument to be moved axially with respect to said surgical instrument and to be rotated with respect to said surgical instrument.

12. The surgical device of claim 11 wherein said surgical instrument includes a surgical implement on a distal end thereof for performing the surgical operation, said coupling being constructed to allow said cannula and said laser instrument to be moved axially between a first position in which said surgical implement extends through said open end of said cannula, and a second position in which said open end of said cannula surrounds said surgical implement.

13. The surgical device of claim 12 wherein said mounting assembly further comprises a frame secured to said cannula, said laser instrument having a proximal end disposed in said frame, said actuator being coupled to said proximal end to move said proximal end axially in said frame and cause said tip of said laser instrument to move axially between said retracted position and said extended position.

14. The surgical device of claim 13 wherein said mounting assembly further comprises
a hollow stem secured to said frame, said surgical instrument extending through said stem into said frame, said stem being received by said coupling,
said coupling including a base for receiving said stem, said stem being axially movable within said base and rotatable within said base.

15. The surgical device of claim 14 further comprising a clamp for securing said stem within said base at a selected axial position and in a selected rotational orientation with respect to said surgical instrument.

16. The surgical device of claim 11 wherein said coupling is configured to threadably to engage said surgical instrument.

17. The surgical device of claim 16 wherein said surgical instrument includes a base from which an elongated tube that supports a surgical implement extends distally through said cannula, said base including an annular collar that is radially spaced from said tube and a set of threads disposed on an interior of said collar,
said coupling having a passage within which said tube is disposed, and having a threaded proximal end configured to fit radially between said collar and said tube and engage said threads.

18. A surgical device comprising
a surgical instrument including a base and an elongated tube that extends distally from said base to a surgical implement for performing a surgical operation on body tissue,
a laser instrument for applying laser energy to body tissue, said laser instrument having a distal tip for emitting the laser energy,
a mounting assembly for attaching said laser instrument to said surgical instrument, said mounting assembly including
an axially elongated cannula within which said tube of said surgical instrument and said laser instrument are disposed, said cannula having an open distal end through which said surgical implement and said tip of said laser instrument can extend,
a frame secured to said cannula, a proximal end of said laser instrument being disposed in said frame,
an actuator for moving said proximal end of said laser instrument axially in said frame and causing said tip of said laser instrument to move axially between a retracted position in which said tip of said laser instrument does not extend distally of said open end of said cannula, and an extended position in which said tip of said laser instrument extends distally of said open end of said cannula, and
a coupling for attaching said cannula to said base, said coupling being constructed to allow said cannula and said laser instrument to be moved axially with respect to said surgical instrument and to be rotated with respect to said surgical instrument.

19. The surgical device of claim 18 further comprising a clamp for securing said cannula to said coupling at a selected axial position and in a selected rotational orientation with respect to said surgical instrument.

20. The surgical device of claim 19 wherein said base includes an annular collar that is radially spaced from said elongated tube and a set of threads disposed on an interior of said collar, said coupling having a passage within which said tube is disposed and having a threaded proximal end configured to fit radially between said collar and said tube and engage said threads.

21. Apparatus for attaching a laser instrument having a tip for applying laser energy to body tissue to a surgical instrument having a surgical implement for performing the surgical operation, said apparatus comprising
a mounting assembly for receiving said laser instrument and constructed for attachment to said surgical instrument, and
an actuator for selectively moving said laser instrument between a retracted position in which said tip of said laser instrument is disposed proximally of said surgical implement, and an extended position in which said tip of said laser instrument is disposed distally of said surgical implement.

22. The apparatus of claim 21 wherein said mounting assembly further comprises
an elongated cannula within which said surgical instrument and said laser instrument are disposed, said cannula having an open distal end through which said surgical implement and said tip of said laser instrument may protrude,
said actuator being disposed on said cannula for selectively moving said laser instrument axially with respect to said cannula between said retracted position and said extended position.

23. The apparatus of claim 22 wherein said mounting assembly further comprises a frame secured to said cannula, a proximal end of said laser instrument being disposed in said frame, said actuator being coupled to said proximal end to move said proximal end axially in said frame and cause said tip of said laser instrument to move axially between said retracted position and said extended position.

24. The apparatus of claim 23 wherein said mounting assembly further comprises a coupling for attaching said cannula to said surgical instrument, said coupling being constructed to allow said cannula and said laser instrument to be moved axially with respect to said surgical instrument and to be rotated with respect to said surgical instrument.

25. The apparatus of claim 24 further comprising a clamp for securing said cannula to said coupling at a selected axial position and in a selected rotational orientation with respect to said surgical instrument.

26. The apparatus of claim 25 wherein said coupling is configured to threadably to engage said surgical instrument.

27. The apparatus of claim 26 wherein said surgical instrument includes a base from which an elongated tube that supports a surgical implement extends distally through said cannula, said base including an annular collar that is radially spaced from said tube and a set of threads disposed on an interior of said collar,
said coupling having a passage within which said tube is disposed, and having a threaded proximal end configured to fit radially between said collar and said tube and engage said threads.

28. The surgical device of claim 1 or 18 wherein said surgical instrument.comprises an arthroscopic surgical instrument.

29. The surgical device of claim 28 wherein said surgical instrument is a powered surgical instrument.

30. The surgical device of claim 1 or 18 wherein the surgical operation includes cutting body tissue.
